Europäisches Patentamt

European Patent Office

Office européen des brevets

(11) Veröffentlichungsnummer : **0 160 340**
**B1**

(12) **EUROPÄISCHE PATENTSCHRIFT**

(45) Veröffentlichungstag der Patentschrift :
07.09.88

(51) Int. Cl.⁴ : **A 61 B   6/00**

(21) Anmeldenummer : 85200612.1

(22) Anmeldetag : 19.04.85

(54) **Röntgengerät mit einem daran verfahrbaren Geräteteil.**

(30) Priorität : 30.04.84 DE 3416000

(43) Veröffentlichungstag der Anmeldung :
06.11.85 Patentblatt 85/45

(45) Bekanntmachung des Hinweises auf die Patenterteilung : 07.09.88 Patentblatt 88/36

(84) Benannte Vertragsstaaten :
**DE FR GB IT**

(56) Entgegenhaltungen :
**FR-A-   324 518**
**FR-A- 1 151 677**
**FR-A- 1 307 731**
**FR-A- 1 520 875**
**FR-A- 1 558 920**
**GB-A- 1 057 600**

(73) Patentinhaber : **Philips Patentverwaltung GmbH**
**Wendenstrasse 35 Postfach 10 51 49**
**D-2000 Hamburg 1 (DE)**
**DE**
**N.V. Philips' Gloeilampenfabrieken**
**Groenewoudseweg 1**
**NL-5621 BA Eindhoven (NL)**
**FR GB IT**

(72) Erfinder : **Kayser, Harald, Dr.**
**Heisterkamp 26**
**D-2000 Wedel (DE)**
Erfinder : **Schmedemann, Walter**
**Dorfstrasse 80**
**D-2000 Tangstedt (DE)**

(74) Vertreter : **Poddig, Dieter, Dipl.-Ing. et al**
**Philips Patentverwaltung GmbH Wendenstrasse 35**
**Postfach 10 51 49**
**D-2000 Hamburg 1 (DE)**

## Beschreibung

Die Erfindung betrifft ein Röntgengerät nach dem oberbegriff des Hauptanspruchs.

Ein solches Röntgengerät ist aus der GB-A 10 57 600 bekannt. Bei dem bekannten Röntgengerät wird jeder Zylinder durch den zugehörigen Kolben in jeweils zwei Teile unterteilt, die bei einer Verschiebung des Kolbens ihr Volumen gegensinnig zueinander ändern. Beide Teile sind mit einer Flüssigkeit gefüllt und über je eine Leitung mit einem korrespondierenden Teil im jeweils anderen Kolben-Zylinder-System verbunden. Dadurch wird erreicht, daß je nach Verschiebungsrichtung des Geräteteils in jeweils einer der Leitungen eine Druckzunahme erfolgt, die das Gegengewicht in die andere Richtung drückt.

Aufgabe der vorliegenden Erfindung ist es, ein Rontgengerät der eingangs genannten Art so auszugestalten, daß sich eine andere Kopplung der beiden Kolben-Zylindereinheiten ergibt die ebenfalls eine leichtgängige Verschiebung des Geräteteils ermöglicht.

Diese Aufgabe wird durch die im Kennzeichen des Anspruchs 1 angegebenen Maßnahmen gelöst. Die zweiseitig ausgebildete Rollmembran dichtet den Flüssigkeitsraum nach außen hermetisch ab. Infolge des großen Durchmesserunterschiedes zwischen Kolben und Zylinder ergibt sich keine Behinderung der Verschiebung durch Reibung. Der Überdruck im Innern der Rollmembran verhindert eine Bildung von zusätzlichen Falten in der Rollmembran, die beim Verschieben einen zusätzlichen Kraftaufwand erfordern würden.

Wenn das Geräteteil bei einem erfindungsgemäßen Röntgengerät bewegt wird, ändert sich das von dem zugehörigen Zylinder und dem zugehörigen Kolben umschlossene Volumen. Diese Volumenänderung erzwingt in dem anderen System aus Zylinder und Kolben eine gleich große aber entgegengesetzte Volumenänderung. Wird beispielsweise in dem einen System das Volumen größer, dann wird es in dem anderen System im gleichen Maße kleiner. Durch diese gegensinnige Änderung der von den Zylindern und den Kolben umschlossenen Flüssigkeitsräume läßt sich eine gegensinnige Bewegung von Geräteteil und Gegengewicht erreichen.

Der Begriff « "Kolben » ist im Zusammenhang mit der Erfindung weit zu interpretieren. Es ist nicht erforderlich, daß der Kolben wie bei der bekannten Einrichtung den Zylinder in zwei Teilräume unterteilt ; vielmehr kann der Kolben auch die Form eines Rohres haben. Wesentlich ist nur, daß der Kolben und der Zylinder zusammen ein Flüssigkeitsvolumen nach außen hin abschließen, wenn man einmal von der darin enthaltenen Öffnung für die hydraulische Verbindung zwischen den beiden aus Zylinder und Kolben bestehenden Systemen absieht.

Bei Rollmembranen wird der äußere Rand am Zylinder eingespannt und durch einen gegenüber dem Zylinder wesentlich kleineren Kolben der Boden der Membran in den übrigen Teil durchgestülpt. Dabei legt sich der annähernd zylindrische Mantel der Membran in dem durchgestülpten Teil an den Kolben, im übrigen Teil an den Zylinder an. Der Zwischenraum wird durch eine sich beim Bewegen abrollende Falte überbrückt. Diese Rollmembranen haben wegen ihrer Herstellung aus ebenem Gewebe eine auf den Durchmesser bezogen beschränkte Höhe und deshalb einen vom Durchmesser abhängigen Hub (Höhe nicht größer als Durchmesser).

Diese Einschränkung wird vermieden, wenn die Membran in rohrförmiger Gestalt mit einer im wesentlichen nur axial oder unter einem spitzen Winkel zur Achse verlaufenden Fadenlage hergestellt wird. Dadurch ist die Elastizität in Umfangsrichtung wesentlich größer als in Längsrichtung. Eine solche Membran wird sich bei Einwirkung einer Druckspannung durch ein fließfähiges Medium von der Innenseite der durchgestülpten Falte her einerseits an den Kolben und andererseits an den Zylinder anlegen, ohne in Längsrichtung auszuweichen. Die Druckspannung bewirkt außerdem, daß beim relativen Verschieben von Kolben und Zylinder die Membran keine die Bewegung behindernden Querfalten bildet.

Im allgemeinen ist als Füllung für die Rollmembran ein flüssiges Medium einem gasförmigen Medium vorzuziehen, weil es damit weniger Diffusionsverluste gibt. Auch hierbei ist ein konstanter Druck, d. h. eine konstante Spannung der Rollenmembran, nicht unter allen Umständen aufrechtzuerhalten ; eine Erwärmung der in der Rollmembran enthaltenen Flüssigkeit könnte infolge ihres thermischen Ausdehnungskoeffizienten zu einer Erhöhung des Druckes führen. Dies läßt sich jedoch nach einer Weiterbildung der Erfindung dadurch vermeiden, daß das Medium mit einem Speicher gekoppelt ist, der durch eine Membran abgeschlossen ist und unter Druck stehendes Gas enthält.

Die Kräfte, die der Benutzer bei einem derart ausgebildeten Röntgengerät aufbringen muß, hängen von der Differenz des Druckes in der Rollenmembran einerseits und in der der Kraftübertragung dienenden Flüssigkeit andererseits ab. Je größer diese Differenz ist, desto mehr Kräfte muß der Benutzer zur Verschiebung des Geräteteils aufbringen. Da bei einem um eine horizontale umlegbaren Röntgenuntersuchungsgerät der Druck in der der Kraftübertragung dienenden Flüssigkeit wie bereits erläutert von der Neigung des Röntgenuntersuchungsgerätes abhängt, ist bei konstantem Druck in dem Druckspeicher — auch die Druckdifferenz bzw. die vom Benutzer zur Verschiebung des Geräteteils aufzubringende Kraft von der jeweiligen Neigung abhängig.

Diese Abhängigkeit läßt sich nach einer Weiterbildung der Erfindung dadurch vermeiden, daß ein durch eine Membran unterteilter Behälter vorgesehen ist, dessen eines Teilvolumen mit der der Kraftübertragung dienenden Flüssigkeit und dessen anderes Teilvolumen mit dem Medium im Innern der Rollmembran in Verbindung steht, und daß Mittel zur Ausübung einer zusätzlichen Kraft auf die Membran vorgesehen sind derart, daß der Druck in dem Medium in der Rollmembran größer ist als der Druck in der der Kraftübertragung dienenden Flüssigkeit. Bei dieser Ausführung wird die Druckdifferenz allein durch die zusätzliche z. B. mittels einer Feder aufgebrachte Kraft auf die Membran hervorgerufen. Durch geeignete Bemessung dieser Kraft läßt sich daher der zum Bewegen des Geräteteils erforderliche Kraftaufwand für den Benutzer besonders klein halten.

Um mit einem Gewichtsausgleich eine bestimmte Verschiebung zwischen Zylinder und Kolben zu erreichen, muß das System Zylinder-Kolben in expandiertem Zustand eine Länge aufweisen, die dem rund 2,5-fachen dieser Verschiebung entspricht. Um nun einerseits die Länge des Systems Zylinder-Kolben nicht zu groß werden zu lassen und andererseits eine ausreichenden Verschiebeweg für das Geräteteil zu erreichen, sieht eine Weiterbildung der Erfindung vor, daß das Geräteteil und/oder das Gegengewicht mit dem zugehörigen Zylinder (Kolben) über eine Hebelanordnung verbunden sind, die so ausgebildet ist, daß bei einer Verschiebung des Geräteteils bzw. des Gegengewichtes der damit gekoppelte Zylinder (Kolben) in geringerem Maße verschoben wird.

Die Erfindung wird nachstehend anhand der Zeichnung näher erläutert. Es zeigen

Fig. 1 ein Ausführungsbeispiel,

Fig. 2a einen Querschnitt durch ein Zylinder-Kolben-System,

Fig. 2b die Erzeugung eines konstanten Innendruckes in der Rollenmembran,

Fig. 2c die Erzeugung einer konstanten Druckdifferenz,

Fig. 3 ein Parallelogrammgestänge zur Umsetzung der Bewegung in eine verringerte Zylinderverschiebung, und

Fig. 4 eine weitere Ausgestaltung des Gerätes nach Fig. 1.

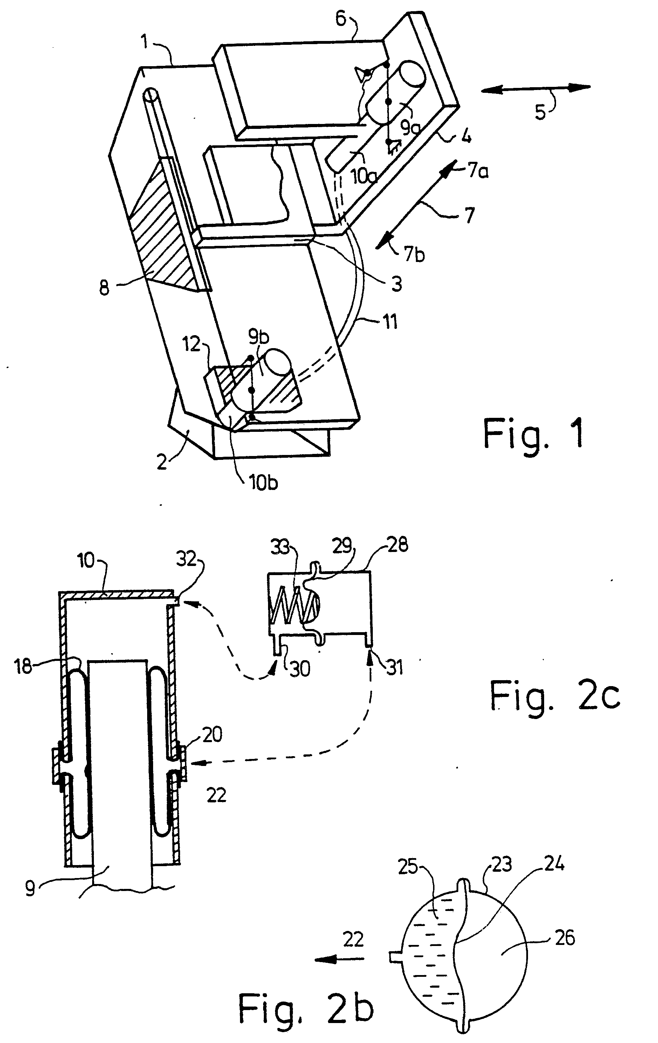

In Fig. 1 ist mit 1 der Rahmen eines Röntgenuntersuchungsgerätes bezeichnet, das an dem Fuß 2 um eine horizontale Achse schwenkbar gelagert ist. Ein Längswagen 3 kann innerhalb des Rahmens 1 in dessen Längsrichtung verschoben werden. Der Längswagen 3 trägt einen Querwagen 4, der auf dem Längswagen quer zur Längsrichtung des Gerätes, d. h. in Richtung des Pfeiles 5, verfahrbar ist. An dem Querwagen 4 ist einerseits eine nicht näher dargestellte Röntgenröhre und andererseits ein Röntgenzielgerät 6 angeordnet, das in Richtung des Pfeiles 7, d. h. senkrecht zu der Längs- und Querrichtung — in der sogenannten Kompressionsrichtung verfahrbar ist.

Das Gewicht des Längswagens mitsamt der von ihm getragenen Bauteile wird mit Hilfe eines in dem Rahmen 1 in dessen Längsrichtung verfahrbaren Hauptgegengewichtes 8 ausgeglichen, das mit dem Längswagen über Seile verbunden ist, die über Rollen geführt sind.

Zum Gewichtsausgleich in Kompressionsrichtung ist das Röntgenzielgerät mit einem Zylinder 9a gekoppelt, der zusammen mit einem an dem Querwagen 4 befestigten Kolben 10a ein Flüssigkeitsvolumen umschließt. Ebenso ist im Fuß des Gerätes ein Kompressionsgegengewicht 12 mit einem Zylinder 9b gekoppelt, der zusammen mit einem Kolben 10b ein Flüssigkeitsvolumen umschließt, das über eine hydraulische Leitung in Form eines Schlauches 11 mit der Flüssigkeit in dem Kolben 10a und im Zylinder 9a kommuniziert. Wenn das Zielgerät 6 in Kompressionsrichtung (Pfeil 7) auf den Rahmen 1 zubewegt wird, senkt sich der Zylinder 9a und das dadurch verdrängte Flüssigkeitsvolumen hebt den Zylinder 9b mit dem damit gekoppelten Gegengewicht 12. Das Produkt aus dem Gewicht 12 und dem Hub dieses Gegengewichtes ist gleich dem Produkt aus dem Gewicht des Zielgerätes 6 und dem Hub des Zielgerätes. Daher besteht ein Gewichtsausgleich : Das Röntgenzielgerät 6 wird durch das Gegengewicht in seiner jeweiligen Position gehalten, und zur Verschiebung des Röntgenzielgerätes muß der Benutzer lediglich Beschleunigungskräfte aufbringen und die Reibung überwinden.

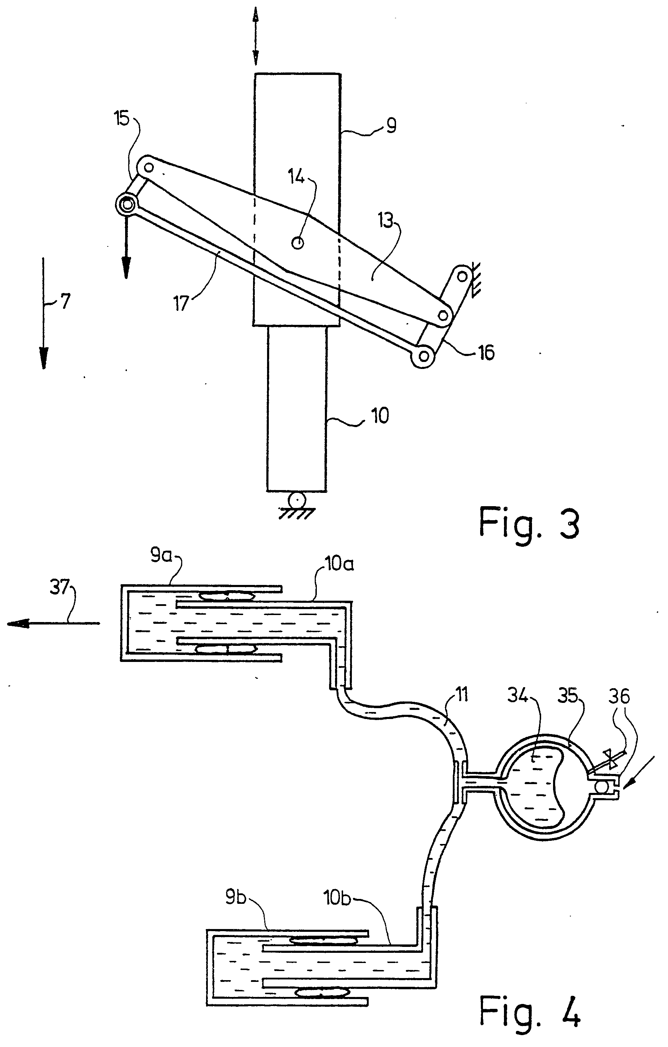

Wie aus Fig. 1 hervorgeht, ist das Zielgerät 6 bzw. das Gegengewicht 12 nicht direkt mit dem zugehörigen Zylinder 9a bzw. 9b gekoppelt, sondern über eine Hebelanordnung in Form eines Parallelogrammgestänges, das den Zylinder 10 auf zwei Seiten umgreift, von denen das eine in Fig. 3 näher dargestellt ist.

Das Parallelogrammgestänge umfaßt einen Hebel 13, der in seiner Mitte bei 14 am Zylinder 9 angelenkt ist und an seinem einen Ende mit dem Ende einer Stange 15 und mit seinem anderen Ende mit der Mitte einer Stange 16 gelenkig verbunden ist. An das andere Ende der Stange 15 ist eine Stange 17 angelenkt, die die gleiche Länge hat wie der Hebel 13 und deren anderes Ende an ein Ende der Stange 16 angelenkt ist derart, daß der Hebel 13 und die Stangen 15, 16 und 17 ein Parallelogramm bilden. Das freie Ende der Stange 16 ist an einem festen Punkt am Querwagen 4 bzw. im Rahmen 1 angelenkt, und zwar so, daß dieser Anlenkpunkt, der Punkt 14 und der Gelenkpunkt zwischen den Stangen 15 und 17 in einer Ebene liegen. An dem Gelenkpunkt der Stangen 15 und 17 ist die Last, d. h. das Zielgerät 6, bzw. das Gegengewicht 12 befestigt. Das Parallelogrammgestänge bewirkt, daß bei einer Verschiebung der Last der Zylinder 9 gegenüber dem feststehenden Kolben 10 den halben Hub erfährt wie die Last. Dadurch kann wie bereits erwähnt die Baulänge des Systems Zylinder 9 — Kolben 10 klein gehalten werden. Durch die beschriebene Ausbildung des Parallelogrammgestänges wird überdies erreicht, daß sich der Gelenkpunkt 14 am Zylinder und der Punkt, an dem die Last angelenkt ist, immer auf parallelen (in Fig. 3 vertikalen) Geraden bewegen, die in Kompressionsrichtung (Pfeil 7) verlaufen.

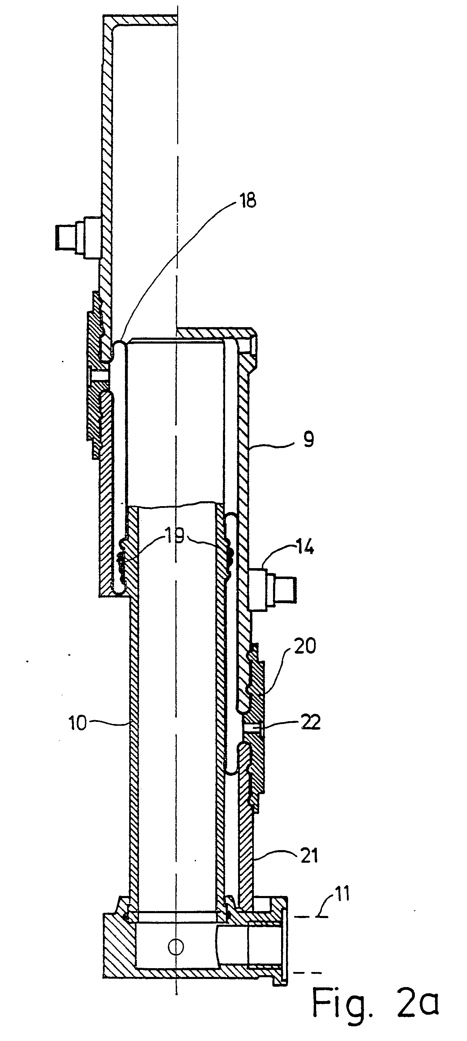

Der Aufbau eines aus Zylinder und Kolben bestehenden Systems ist in Fig. 2a näher dargestellt, wobei das System rechts von der Mittellinie in ganz zusammengedrücktem und links von der Mittellinie in ganz auseinandergezogenem Zustand dargestellt ist. Der Kolben 10 besteht aus einem Rohr, das an seinem unteren Ende mit einem Anschluß für den Schlauch 11 (Fig. 1) versehen ist. Der Zylinder 9, der den Kolben 10 umschließt, ist an seinem oberen Ende durch eine Stirnfläche abgeschlossen. Sein Innendurchmesser (z. B. 62 mm) ist deutlich größer als der Außendurchmesser des Kolbens 10 (z. B. 46 mm), so daß zwischen Kolben und Zylinder ein relativ großer Zwischenraum verbleibt. Dieser wird mit Hilfe einer zweiseitigen Rollmembran 18 abgedichtet, die den Kolben 10 umschließt. Die Mitte dieser Rollmembran ist bei 19 auf dem ganzen Umfang mit dem Kolben verbunden. Ihre Enden sind mit Hilfe eines Preßringes 20 mit dem unteren Ende des Zylinders 9 sowie mit einem Zylinderteil 21 verbunden, der nur als Führungselement dient und dessen Innen- und Außendurchmesser mit den entsprechenden Maßen des Zylinders 9 übereinstimmen. Die zweiseitige Rollenmembran 18 dichtet also einerseits den Zwischenraum zwischen dem Zylinder 9 und dem Kolben 10 ab und begrenzt andererseits ein vorzugsweise flüssiges Medium, z. B. Glycerin.

Der Druck in dieser Flüssigkeit ist größer als der Druck in der der Kraftübertragung dienenden Flüssigkeit, deren Volumen durch den Zylinder 9 einschließlich seiner Stirnfläche die eine Seite der Rollmembran 18 und den Kolben 10 begrenzt wird. Infolgedessen bläht sich die Rollenmembran nach außen hin auf und wälzt sich bei einer Verschiebung des Zylinders auf dessen Innenflächen sowie auf der Außenfläche des Kolbens faltenfrei ab. Durch den Druck im Innern der Rollmembran wird außerdem erreicht, daß der Zylinder 9 koaxial zum Kolben 10 ausgerichtet wird.

Wäre der Innendruck in der Rollmembran kleiner als der Druck in der der Kraftübertragung dienenden Flüssigkeit, würde die Rollmembran auf ihrer dieser Flüssigkeit zugewandten Seite scharfe Falten bilden, die eine Verschiebung zwischen Kolben und Zylinder praktisch blockieren würden.

Die Rollmembran, deren Elastizität zumindest in axialer Richtung möglichst gering sein sollte, kann aus einem Gewebe von in axialer Richtung verlaufenden Fäden bestehen, das beidseitig durch eine Gummihaut abgedichtet ist. Je dünner die Rollmembran ist, desto geringer sind die bei einer Verschiebung des Zylinders auftretenden Walkverluste in der Membran und damit letztlich die vom Benutzer zur Bewegung des Zielgerätes aufzubringenden Kräfte. Die Walkverluste werden aber auch durch den Durchmesser der Innenfläche des Zylinders einerseits und der Außenfläche des Kolbens andererseits bestimmt. Je größer der Durchmesserunterschied ist, desto größer ist der Durchmesser der Krempelfalte der Rollmembran und desto kleiner sind die Walkverluste. Auf der anderen Seite steigt die Belastung des Gewebes mit der Größe der Kreisringfläche zwischen Kolben und Zylinder, so daß bei größeren Durchmesserunterschieden dickere Rollmembranen erforderlich sein können.

Wie bereits erwähnt, muß der Innendruck in der Rollmembran größer sein als der Druck in der Flüssigkeit zur Kraftübertragung. Dieser erhöhte Innendruck kann dadurch aufrechterhalten werden, daß das Innere der Rollmembran hermetisch nach außen abgedichtet wird. Allerdings können sich auch dann noch aufgrund thermischer Ausdehnungungen und der geringen Nachgiebigkeit der Rollmembran unerwünschte Druckschwankungen ergeben. Diese Druckschwankungen können aber vermieden werden, wenn das Innere der Rollmembran über eine in dem Preßring 20 vorgesehene Öffnung 22 an einen Druckspeicher angeschlossen wird. Dieser Druckspeicher ist in Fig. 2 b schematisch dargestellt. Er besteht aus einem Metallgefäß 23, das durch eine elastische Membran 24 in zwei Kammern unterteilt wird, von denen die Kammer 25 mit der auch in der Rollmembran befindlichen Flüssigkeit gefüllt ist und über den Auslaß 22 mit dieser in Verbindung steht, während die zweite nach außen hin hermetisch dichte Kammer ein unter Druck stehendes Gas z. B. Stickstoff enthält.

Die Walkverluste sind um so größer, je größer der Druckunterschied zwischen der Flüssigkeit in der Rollmembran und dem Atmosphärendruck einerseits bzw. dem Druck in der der Kraftübertragung dienenden Flüssigkeit andererseits ist. Die größten Walkverluste ergeben sich daher bei einer Ausführungsform mit konstantem Druck in der Rollmembran, wenn der Tisch des Röntgenuntersuchungsgerätes senkrecht steht, weil dann der Druck in der der Kraftübertragung dienenden Flüssigkeit praktisch Null ist, so daß sich eine große Druckdifferenz ergibt. Dieser Nachteil läßt sich vermeiden, wenn anstatt eines ständig einen konstanten (maximalen) Druck erzeugenden Speichers ein Druckerzeuger tritt, der immer nur die zur Aufrechterhaltung der Spannung der Rollenmembran erforderliche Druckdifferenz liefert. Dieser in Fig. 2c dargestellte Druckerzeuger besteht aus einem Gefäß 28, das wiederum durch eine Membran 29 in zwei Teile unterteilt wird, von denen jeder einen Auslaß 30 bzw. 31 besitzt. Der Auslaß 31 ist mit dem Auslaß 22 und der Auslaß 30 mit einem Auslaß 32 in der Nähe der Stirnfläche des Zylinders 10 über je einen nicht näher dargestellten Schlauch verbunden. In der mit dem Auslaß 30 bzw. über eine nicht näher dargestellte Leitung mit dem Auslaß 32 im Zylinder 10 verbundenen Kammer ist zusätzlich eine Druckfeder 33 vorgesehen, die auf einen Teil der Membranfläche einen Druck ausübt. Ohne diese Feder würde diese Membran stets eine solche Lage einnehmen, daß der Druck im Innern der Rollmembran gleich dem Druck in der der Kraftübertragung dienenden Flüssigkeit wäre. Durch die Feder 33 jedoch wird der Druck auf die mit dem Auslaß 31 versehene Kammer erhöht, so daß der Druck in der Rollmembran um einen vorgegebenen, von der Federkraft abhängigen Wert größer ist als der Druck in der der Kraftübertragung dienenden Flüssigkeit.

Im Falle eines Lecks in der Rollenmembran würde die Membran 29 durch die Federkraft ganz nach rechts verschoben. Wenn dort ein geeigneter Schalter angeordnet wird, z. B. ein Reed-Kontakt, der durch einen Magneten an der in die Extremlage geschobene Membran betätigt wird, kann ein Alarmsignal erzeugt werden.

Wie bereits erwähnt, verschwindet der Druck in der der Kraftübertragung dienenden Flüssigkeit, wenn das Röntgenuntersuchungsgerät in seine senkrechte Stellung geschwenkt ist. Wenn in dieser Stellung mit geringem Kraftaufwand am Röntgenzielgerät gezogen wird, kann das Gegengewicht dieser Bewegung folgen. Übersteigt die am Zielgerät 6 angreifende Kraft jedoch einen kritischen Wert, der dem Produkt aus dem Atmosphärendruck und der wirksamen Kolbenfläche entspricht, dann entsteht ein Vakuum bzw. ein Unterdruck, so daß in der Flüssigkeit Dampf gebildet wird ; das Gegengewicht bewegt sich dann nicht genauso schnell wie das Zielgerät, sondern folgt verzögert und bewirkt beim Zusammenfall der Dampfblase einen störend spürbaren Schlag.

Die erwünschte Funktion des Gegengewichts ist nur, das Röntgenzielgerät 6 in Richtung 7a (aufwärts) zu stützen. Eine Kraftwirkung entgegen dieser Richtung ist nicht erforderlich und ihr Wegfall ist sogar ein Vorteil bei der Bedienung am senkrecht stehenden Gerät, weil beim Bewegen des Röntgenzielgerätes in Richtung 7 das Gegengewicht 12, wenn es einmal in seine Endlage gefahren ist, nicht an der Bewegung teilnimmt und deshalb auch keine von der Bedienung aufzubringenden Beschleunigungskräfte erfordert.

Diese Entkopplung in einer Richtung ist in Fig. 4 gezeigt, womit einerseits der erwähnte Bedienungsvorteil, andererseits der Wegfall des weiter oben aufgeführten Schlages nach Vakuumbildung erreicht wird. Danach steht der Schlauch 11 in Verbindung mit elastischen Blase 34, die sich im Innern eines starren Gehäuses 35 befindet, das mit einer Öffnung oder mit einer Ventilanordnung 36 versehen ist, so daß Luft in das Gehäuse hinein und — gegebenenfalls gedämpft — aus dem Gehäuse herausströmen kann. Wird nun eine Kraft in Richtung des Pfeiles 37 bzw. 7a, d. h. eine Zugkraft auf das Zielgerät 6 bzw. den damit verbundenen Zylinder 9a, ausgeübt, folgt der Zylinder 9a dieser Kraft, wobei das sich vergrößernde Volumen im System 9a, 10a durch die aus der elastischen Blase 34 nachströmende Flüssigkeit gefüllt wird. Der Zylinder 9b verschiebt sich dabei nicht gegenüber dem Zylinder 10b, d. h. das Gegengewicht ist dann nicht mehr mit den Bewegungen des Zielgerätes gekoppelt, was wie erwähnt den Vorteil hat, daß in dieser Stellung der Benutzer nur die Masse des Zielgerätes bewegen muß.

Wenn das Röntgenuntersuchungsgerät aus der senkrechten Stellung geschwenkt wird, gerät die Flüssigkeit wieder unter Druck, wodurch die elastische Blase 34 sich ausdehnt und damit die eingeschlossene Luft verdrängt. Gleichzeitig wird das Ventil 36 geschlossen oder nur mit einem kleinen Drosselquerschnitt geöffnet gehalten, so daß die Luft nur langsam ausströmen kann, bis die elastische Blase 34 sich überall an die Innenwände des Gehäuses 35 anschmiegt. Dadurch erfolgt die Kopplung nicht schlagartig, sondern sanft. Danach sind die Bewegungen der Zylinder 9a und 9b wieder miteinander gekoppelt.

Es ist nicht unbedingt erforderlich, daß die beiden mit dem Zielgerät 6 bzw. dem Gegengewicht 12 verbundenen Zylinder-Kolben-Systeme identische Abmessungen aufweisen. Bei unterschiedlichen Abmessungen beider Systeme lassen sich unterschiedliche Übersetzungsverhältnisse erreichen (d. h. daß sich dann bei einer Verschiebung des Zylinders 9a der Zylinder 9b nicht um die gleiche Strecke verschiebt), was ggf. benutzt werden kann, um mit kleineren Gegengewichten oder mit kleineren Zylinderhüben den Gewichtsausgleich zu erreichen.

Es ist auch nicht wichtig, daß der Zylinder den Kolben umschließt. Vielmehr könnte der Zylinder im Innern des rohrförmigen Kolbens angeordnet und über eine Rollmembran mit dem Kolben verbunden sein.

Zur Verringerung der Baulänge könnten in einem Zylinder auch zwei Kolben vorzugsweise mit unterschiedlichem Durchmesser aus entgegengesetzten Richtungen eintauchen, so daß sie ineinander eindringen können. Die Kolben müßten dann an beiden Seiten durch je eine Stirnfläche abgeschlossen sein und der Zylinderdurchmesser müßte in axialer Richtung so angepaßt werden, daß die Mitte zwischen der Zylinderinnenwand und der Kolbenaußenwand jeweils den gleichen Abstand von der Zylinderachse aufweist. Statt eines aus nur zwei Teilen bestehenden Zylinder-Kolben-Systems könnte auch ein System mit mehr als zwei zusammenschiebbaren Teilen mit gestuften Durchmessern verwendet werden, zwischen denen jeweils eine Rollmembran angeordnet wäre. Diese Teile würden dann bei einer Verschiebung teleskopartig auseinander- oder zusammengeschoben, wobei durch geeignete Wahl der Durchmesser eine gleichmäßige synchrone Verschiebung aller Teleskopteile erreichbar ist.

**Patentansprüche**

1. Röntgengerät mit einem daran verfahrbaren Geräteteil (6) und einem damit hydraulisch gekoppelten gegensinnig zu dem Geräteteil bewegbaren Gegengewicht (12), wobei dem Geräteteil und dem Gegengewicht je ein Zylinder (9a, 9b) und ein Kolben (10a, 10b) zugeordnet sind, von denen der eine Teil fest und der andere Teil mit dem Geräteteil (6) bzw. dem Gegengewicht (12) verbunden ist, und wobei die durch je einen Kolben und einen Zylinder gebildeten Flüssigkeitsräume hydraulisch miteinander gekoppelt sind, dadurch gekennzeichnet, daß zwischen den beiden Kolben-Zylindereinheiten nur eine einzige Verbindung (11) vorgesehen ist, daß der Außendurchmesser jedes der beiden Kolben (10a, 10b) deutlich kleiner als der Innendurchmesser des zuge-

hörigen Zylinders (9a, 9b) ist und daß der Zwischenraum durch eine zweiseitig ausgebildete Rollmembran (18) abgedichtet ist, die auf ihrem Umfang mit dem Kolben sowie mit dem Zylinder verbunden ist und die mit einem flüssigen oder gasförmigen Medium gefüllt ist, dessen Innendruck größer ist als der Druck in dem Flüssigkeitsraum.

2. Röntgengerät nach Anspruch 1, dadurch gekennzeichnet, daß das flüssige oder gasförmige Medium mit einem Druckspeicher (23,) gekoppelt ist, der durch eine Membran abgeschlossen ist und unter Druck stehendes Gas enthält (Fig. 2b).

3. Röntgengerät nach Anspruch 1, dadurch gekennzeichnet, daß ein durch eine Membran (29) unterteilter Behälter (25) vorgesehen ist, dessen eines Teilvolumen mit der der Kraftübertragung dienenden Flüssigkeit und dessen anderes Teilvolumen mit dem Medium im Innern der Rollmembran (18) in Verbindung steht, und daß Mittel (33) zur Ausübung einer zusätzlichen Kraft auf die Membran vorgesehen sind derart, daß der Druck in dem Medium in der Rollmembran größer ist als der Druck in der der Kraftübertragung dienenden Flüssigkeit (Fig. 2c).

4. Röntgengerät nach einem der vorhergehenden Ansprüche, dadurch gekennzeichnet, daß mit der hydraulischen Verbindung (11) bzw. mit dem Arbeitsraum in den beiden Zylindern ein Behälter mit veränderbarem Volumen gekoppelt ist.

5. Röntgengerät nach Anspruch 4, dadurch gekennzeichnet, daß eine mit der Flüssigkeit in den Zylindern in Verbindung stehende elastische Blase (34) im Innern eines starren Gehäuses (35) angeordnet ist, das mit einer Öffnung versehen ist, so daß der atmosphärische Druck auf die Blase mit veränderbarem Volumen einwirken kann.

6. Röntgengerät nach Anspruch 5, dadurch gekennzeichnet, daß das Gehäuse mit einem Ventil (36) versehen ist, durch das die Luft aus dem Raum zwischen den beiden Behältern wahlweise gesteuert ungedämpft oder gedämpft ausströmen kann.

7. Röntgengerät nach einem der vorhergehenden Ansprüche, dadurch gekennzeichnet, daß das Geräteteil (6) und/oder das Gegengewicht (12) mit dem zugehörigen Zylinder (9a, 9b) (Kolben) über eine Hebelanordnung (13...17) verbunden ist, die so ausgebildet ist, daß bei einer Verschiebung des Geräteteils (6) bzw. des Gegengewichtes (12) der damit gekoppelte Zylinder (9a, 9b) (Kolben) in geringerem Maße verschoben wird.

## Claims

1. An X-ray apparatus provided with a component (6) slidably carried thereon and a counterweight (12) which is hydraulically coupled thereto and which is displaceable in the opposite sense with respect to the component, a respective cylinder (9a, 9b) and a respective piston (10a, 10b) being associated with the component and the counterweight, one part thereof being stationary whilst the other part is connected to the component (6) and the counterweight (12), respectively, the liquid spaces formed by a respective piston and a cylinder being hydraulically coupled to one another, characterized in that between the two piston/cylinder units there is provided only a single connection (11), the outer diameter of each of the two pistons (10a, 10b) being substantially smaller than the inner diameter of the associated cylinder (9a, 9b), the intermediate space being sealed by a double roll diaphragm (18) whose periphery is connected to the piston as well as to the cylinder and which is filled with a liquid or gaseous medium whose inner pressure exceeds the pressure in the liquid space.

2. An X-ray apparatus as claimed in Claim 1, characterized in that the liquid or gaseous medium is coupled to a pressure reservoir (23) which is sealed by a diaphragm and which contains pressurized gas (Fig. 2b).

3. An X-ray apparatus as claimed in Claim 1, characterized in that there is provided a container (28) which is subdivided by a diaphragm (29) and one sub-volume of which communicates with the force transferring liquid whilst its other sub-volume communication with the medium inside the roll diaphragm (18), there also being provided means (33) for exerting an additional force on the diaphragm so that the pressure in the medium inside the roll diaphragm exceeds the pressure in the force transferring liquid (Fig. 2c).

4. An X-ray apparatus as claimed in any one of the preceding Claims, characterized in that a container of variable volume is coupled to the hydraulic connection (11) or to the working space inn the two cylinders.

5. An X-ray apparatus as claimed in Claim 4, characterized in that an elastic bladder (34) which communicates with the liquid in the cylinders is arranged inside a rigid housing (35), which bladder is provided with an opening so that atmospheric pressure can act on the bladder of variable volume.

6. An X-ray apparatus as claimed in Claim 5, characterized in that the housing is provided with a valve (36) which enables the controlled outflow of the air from the space between the two containers in a non-damped or damped manner, as desired.

7. An X-ray apparatus as claimed in any one of the preceding claims, characterized in that the component (6) and/or the counterweight (12) is connected to the associated cylinder (9a, 9b) (piston) via a system of rods (13...17) which is constructed so that when the component (6) or the counterweight (12) is displaced, the cylinder (9a, 9b) (piston) is displaced to a lesser extent.

## Revendications

1. Appareil à rayons x comportant une partie d'appareil (6) déplaçable et un contrepoids (12) couplé hydrauliquement à la partie d'appareil et mobile dans un sens opposé par rapport à celle-ci, un cylindre (9a, 9b) et un piston (10a, 10b) étant associés respectivement à la partie d'appareil et au contrepoids, l'un de ces éléments étant fixe et l'autre étant relié à la partie d'appareil (6) et au contrepoids (12), les chambres de liquide formées chaque fois par un piston et un cylindre étant couplées hydrauliquement l'une à l'autre, caractérisé en ce qu'entre les deux unités à piston et cylindre n'est prévue qu'une seule liaison (11), le diamètre extérieur de chacun des deux pistons (10a, 10b) est nettement plus petit que le diamètre intérieur du cylindre (9a, 9b) associé et l'espace qui les sépare est rendu étanche par une membrane roulante de configuration bilatérale (18) qui, sur son pourtour, est reliée au piston et au cylindre et qui est remplie d'un milieu liquide ou gazeux dont la pression interne est supérieure à la pression régnant dans la chambre de liquide.

2. Appareil à rayons x suivant la revendication 1, caractérisé en ce que le milieu liquide ou gazeux est couplé à un accumulateur de pression (23) qui est fermé par une membrane et qui contient du gaz sous pression (Fig. 2b).

3. Appareil à rayons X suivant la revendication 1, caractérisé en ce qu'un récipient (28) divisé par une membrane (29) est prévu, son premier volume partiel communiquant avec le liquide servant à transmettre les forces et son autre volume partiel communiquant avec le milieu présent à l'intérieur de la membrane roulante (18), et des moyens (33) destinés à exercer une force supplémentaire sur la membrane sont prévus de telle façon que la pression régnant dans le milieu présent dans la membrane roulante soit supérieure à la pression régnant dans le liquide servant à transmettre les forces (Fig. 2c).

4. Appareil à rayons X suivant l'une quelconque des revendications précédentes, caractérisé en ce qu'un récipient à volume variable est couplé à la liaison hydraulique (11) ou à la chambre de travail dans les deux cylindres.

5. Appareil à rayons X suivant la revendication 4, caractérisé en ce qu'une vessie élastique (34) en communication avec le liquide contenu dans les cylindres est prévue à l'intérieur q'une capacité rigide (35) qui est pourvue d'une ouverture, de sorte que la pression atmosphérique peut agir sur la vessie de volume variable.

6. Appareil à rayons X suivant la revendication 5, caractérisé en ce que la capacité est pourvue d'une valve (36) par laquelle l'air peut s'échapper de l'espace entre les deux récipients d'une manière, au choix, étranglée ou non étranglée.

7. Appareil à rayons X suivant l'une quelconque des revendications précédentes, caractérisé en ce que la partie d'appareil (6) et/ou le contrepoids (12) sont reliés aux cylindres associés (9a, 9b) (piston) par l'intermédiaire d'un système de leviers (13,. ..., 17) qui est agencé de telle façon que, lors d'un déplacement de la partie d'appareil (6) ou du contrepoids (12), le cylindre (9a, 9b) (piston) qui y est couplé soit déplacé dans une mesure plus faible.

# 0 160 340

Fig. 1

Fig. 2c

Fig. 2b

0 160 340

Fig. 2a

2

Fig. 3

Fig. 4

3